# FASCICULE DE BREVET EUROPEEN

(11) **EP 2 598 109 B1**
(45) Date de publication et mention de la délivrance du brevet: **11.07.2018**
(21) Numéro de dépôt: 11736108.9
(22) Date de dépôt: 28.07.2011
(51) Int. Cl.: A61P 27/04, A61P 17/00, A61K 36/00, A61Q 19/00, A61K 31/7016, A61K 31/728, A61K 31/07, A61K 31/70, A61K 31/4164, A61K 8/60, A61K 8/97, A61K 8/49, A61K 8/34, A61K 45/06, A61K 8/73

(54) **COMPOSITION A USAGE TOPIQUE SANS CONSERVATEUR COMPRENANT DE L'ACIDE HYALURONIQUE**
ZUSAMMENSETZUNG OHNE KONSERVIERUNGSSTOFFE ZUR TOPISCHEN VERWENDUNG MIT HYALURONSÄURE
PRESERVATIVE-FREE COMPOSITION FOR TOPICAL USE INCLUDING HYALURONIC ACID

(30) Priorité: 28.07.2010 FR 1056221
(43) Date de publication de la demande: 05.06.2013
(73) Titulaire: Horus Pharma, 06700 Saint-Laurent-du-Var (FR)
(72) Inventeur: CLARET, Claude, F-06100 Nice (FR); CLARET, Martine, F-06100 Nice (FR); GARD, Carole, F-06110 La Cannet (FR)
(74) Mandataire: Regimbeau
(86) Numéro de dépôt international: PCT/EP2011/062971
(87) Numéro de publication internationale: WO 2012/013736

(56) Documents cités:
- WO-A1-2009/044423
- WO-A1-2009/113820
- WO-A1-2010/003797
- FR-A1- 2 919 185
- US-A1- 2006 182 794
- DATABASE WPI Week 201018 Thomson Scientific, London, GB; AN 2010-B20094 XP002626138, & CN 101 627 956 A (SUN H) 20 janvier 2010 (2010-01-20)
- DATABASE WPI Week 201017 Thomson Scientific, London, GB; AN 2010-B20092 XP002626139, & CN 101 627 958 A (SUN H) 20 janvier 2010 (2010-01-20)
- DATABASE WPI Week 200912 Thomson Scientific, London, GB; AN 2009-E79265 XP002626151, & KR 2008 082 122 A (JAE H K) 11 septembre 2008 (2008-09-11)
- DATABASE WPI Week 200682 Thomson Scientific, London, GB; AN 2006-811405 XP002626140, -& RU 2 277 954 C2 (VESELEVA N I) 20 juin 2006 (2006-06-20)
- Horus Pharma: "ilast Post-op", , juin 2010 (2010-06), XP002626141, Extrait de l'Internet: URL:http://www.horus-pharma.com/images/sto ries/files/ilast_post_op_notice.pdf [extrait le 2011-03-02]
- Horus Pharma: "ilast Hydra", , juin 2010 (2010-06), XP002626142, Extrait de l'Internet: URL:http://www.horus-pharma.com/index.php/ fr/produits/dermatologie/104-ilast-hydra [extrait le 2011-03-02]
- Horus Pharma: "ilast Care", , juin 2010 (2010-06), XP002626143, Extrait de l'Internet: URL:http://www.horus-pharma.com/index.php/ fr/produits/dermatologie/105-ilast-care [extrait le 2011-03-02]
- TRB Chemedica: "VISMED gel", , octobre 2003 (2003-10), XP002626144, Extrait de l'Internet: URL:http://www.horus-pharma.fr/images/stor ies/files/vismed_gel_notice.pdf [extrait le 2011-03-02]
- TRB Chemedica: "VISMED multi", , mai 2009 (2009-05), XP002626145, Extrait de l'Internet: URL:http://www.horus-pharma.fr/images/stor ies/files/vismed_multi_notice.pdf [extrait le 2011-03-02]
- DATABASE WPI Week 199445 Thomson Scientific, London, GB; AN 1994-364368 XP002659678, & KR 940 000 326 B1 (KIM T) 17 janvier 1994 (1994-01-17)

## Description

La présente invention concerne des compositions à usage topique comprenant de l'acide hyaluronique, dépourvues de conservateurs, leurs utilisations et un procédé permettant de les obtenir.

La composition peut tout particulièrement être destinée à une application topique, en particulier sur des peaux et/ou des muqueuses fragiles, en particulier celles protégeant l'oeil.

Les sourcils, les cils et les paupières sont des éléments primordiaux du système de protection de l'oeil. En particulier les paupières constituent une barrière protectrice des globes oculaires contre les agressions externes. La peau qui recouvre les paupières est très sensible, très fine et requiert d'être relativement bien hydratée.

La perméabilité de la peau a été largement étudiée en dermatologie et dépend de nombreux facteurs, dont l'épaisseur et le degré d'hydratation. Ainsi, toute composition appliquée sur les tissus à proximité du globe oculaire sera susceptible de pénétrer également dans l'oeil.

Ceci est confirmé par les rares études relatives à l'anesthésie locale des paupières à l'aide de crème EMLA, lidocaïne et prilocaïne, et la toxicité oculaire induite, ainsi que par les conséquences oculaires de l'utilisation de certains cosmétiques pour maquillage, comme le khôl, le mascara, etc.

Par ailleurs, la conjonctive qui fait partie de la paupière, conjonctive palpébrale, et qui recouvre la partie antérieure de l'oeil, est sensible aux effets indésirables des conservateurs présents dans les compositions, comme le BAK ou le Thiomersal. En effet, ceux-ci peuvent entraîner différents effets indésirables, comme une hyperhémie conjonctivale et une réaction allergique.

Il est ainsi souhaitable de disposer de compositions dépourvues ou limitant ces effets indésirables tout en permettant une utilisation topique sur la peau et/ou les muqueuses fragiles et/ou lésées.

L'acide hyaluronique est un composé majeur de la peau. Il est notamment utile dans la réparation des peaux et des muqueuses lésées.

Les compositions à usage topique existantes comprenant de l'acide hyaluronique présentent en général une teneur en celui-ci relativement faible, des acides hyaluroniques de poids moléculaire insuffisant, une viscosité insatisfaisante, de nombreux composés et agents de texture, des conservateurs chimiques et substances aux propriétés antiseptiques ou tensioactives éventuellement non-compatibles ou susceptibles de provoquer des effets irritants ou indésirables avec les muqueuses et/ou la peau, en particulier certaines peaux lésées, les tissus oculaires et ses annexes.

En particulier dans le cas d'une utilisation topique de ces compositions sur des peaux ou des muqueuses fragiles, les compositions de l'art antérieur peuvent présenter l'inconvénient d'un lavage et/ou d'une protection, par exemple des paupières, insuffisants, d'une prévention et/ou d'un traitement, notamment de la sécheresse oculaire, insatisfaisants, et/ou des problèmes d'allergisation, par exemple dû à la présence de multiples composés.

Les compositions de l'art antérieur peuvent également comprendre un grand nombre de composés. Ce grand nombre de composés peut conduire à des « synergies » indésirables, une cytotoxicité trop importante, notamment liée à la présence de conservateurs, et des composés antiseptiques, comme des huiles essentielles, peuvent présenter des risques d'effets indésirables. Ce type d'effet indésirable peut être particulièrement notable sur certaines parties des tissus cutanés, comme par exemple les tissus du visage et des mains, exposés plus fréquemment aux agressions environnementales, ou les tissus des paupières.

Le document CN101627958 décrit un procédé de préparation d'une composition cosmétique comprenant de l'acide hyaluronique et des conservateurs. Le document KR20080082122 décrit une composition thérapeutique stérile, à usage topique, dépourvue de conservateurs mais comprenant plus de 10% d'extrait végétal.

Il est donc souhaitable d'obtenir une composition présentant une bonne efficacité, en particulier en termes énoncés ci-dessus, par exemple de lavage, de protection, de prévention et/ou de traitement, tout en ayant un minimum d'effets indésirables, notamment tels que ceux énoncés ci-dessus.

Par ailleurs, les procédés permettant de stériliser ou de décontaminer une formule, comme la stérilisation chimique, via l'utilisation d'agents chimiques et/ou de conservateurs dans la formule, la stérilisation ionisante ou la stérilisation par chauffage présentent un certain nombre d'inconvénients notamment dans le cas de la préparation d'une composition comprenant de l'acide hyaluronique, en particulier quand celui-ci est présent en une teneur relativement importante et/ou présente un haut poids moléculaire.

Par exemple, la stérilisation chimique est peu, voire pas, compatible avec une utilisation de la composition sur des tissus ou des muqueuses lésés.

La stérilisation ionisante peut pour sa part entraîner une altération partielle, voire une destruction totale, de certains des produits entrant dans la composition, dont en particulier l'acide hyaluronique.

Les procédés de décontamination ou de stérilisation comprenant une stérilisation par chauffage du produit à des températures supérieures ou égales à 120°C, peuvent affecter l'intégrité du conditionnement et/ou les propriétés des ingrédients.

Enfin, lorsque des températures de décontamination ou de stérilisation basses, notamment inférieure ou égales à 80°C, sont utilisées il est généralement nécessaire de conserver ensuite le produit au froid. En conséquence, le produit obtenu présente souvent une durée de conservation courte.

Ainsi, la présente invention vise à permettre de résoudre les problèmes évoqués ci-dessus en tout ou partie. En particulier l'invention vise à fournir une composition présentant une bonne efficacité, simple à obtenir, ayant peu ou pas d'effets indésirables, une durée et des conditions de conservation satisfaisantes, notamment à température ambiante, et un procédé simple permettant d'obtenir une telle composition.

Selon un premier aspect, la présente invention a pour objet une composition stérile et/ou décontaminée avec un dénombrement de la flore aérobie inférieur à 1 CFU/ml, à usage topique comprenant, ou consistant en :
- de l'acide hyaluronique à une teneur supérieure ou égale à 0,1 % en poids par rapport au poids total de la composition, et
- au moins un agent de cicatrisation de la peau choisi parmi l'allantoïne, la vitamine A, le sucralfate et le saccharose sulfate basique d'aluminium;
- éventuellement un extrait de plante, notamment ayant des propriétés médicinales et/ou pharmaceutiques en une quantité inférieure à 10% en poids et
- au moins un solvant.

Par « décontaminée », on entend au sens de la présente invention que les contrôles de propreté microbiologique sont effectués suivant les critères de la norme ISO 21149, soit par dénombrement de la flore aérobie (inférieure à 1CFU/ml), l'absence de pseudomonas aeruginosa, l'absence de staphylococcus aureus et l'absence de candida albicans.

Par « stérile », on entend au sens de la présente invention l'absence de germes au sens de la Pharmacopée Européenne, 6^{ème} édition - 2008.

L'acide hyaluronique est un polymère de disaccharide, formé d'acide D-glucuronique et d'une molécule de N-acétyl-glucosamine.

Il est présent naturellement dans de nombreux tissus, en grande partie dans la peau, en particulier dans l'épiderme, ainsi que dans les tissus conjonctifs et représente l'un des principaux constituants de la matrice extracellulaire. La longueur de la molécule varie selon les tissus, l'espèce et l'état du tissu.

L'acide hyaluronique peut être obtenu par extraction tissulaire à partir de tissus animaux ou par fermentation bactérienne, notamment avec streptococcus equi ou bacillus subtilis.

La composition comprend de l'acide hyaluronique à une teneur supérieure ou égale à 0,1 % en poids, voire une teneur supérieure ou égale à 0,2 % en poids, notamment à une teneur supérieure ou égale à 0,3 % en poids, en particulier supérieure ou égale à 0,4 % en poids, voire supérieure ou égale à 0,5 % en poids par rapport au poids total de la composition.

Tout particulièrement la teneur en acide hyaluronique va de 0,1 à 2 % en poids, en particulier de 0,2 à 2 % en poids, notamment de 0,2 à 1 % en poids, tout particulièrement de 0,2 à 0,5 % en poids, par rapport au poids total de la composition.

Selon un mode de réalisation particulier, la composition comprend une teneur en acide hyaluronique allant de 0,1 à 1 % en poids, en particulier de 0,1 à 0,5 % en poids par rapport au poids total de la composition

L'acide hyaluronique peut être un mélange d'acide hyaluronique de bas poids moléculaire et d'acide hyaluronique de haut poids moléculaire.

L'acide hyaluronique de haut poids moléculaire peut avoir un poids moléculaire allant de 10 à 1 200 kDa, notamment de 10 à 1 000 kDa, en particulier de 10 à 800 kDa.

Tout particulièrement, l'acide hyaluronique de haut poids moléculaire a un poids moléculaire allant de 600 à 1 200 kDa, notamment de 800 à 1 200 kDa.

L'acide hyaluronique de bas poids moléculaire peut avoir un poids moléculaire allant de 10 à 1 000 kDa, notamment de 10 à 600 kDa.

Tout particulièrement, l'acide hyaluronique de bas poids moléculaire a un poids moléculaire allant de 400 à 1 000 kDa, notamment de 600 à 1 000 kDa.

Lorsque la composition comprend à la fois de l'acide hyaluronique de haut et de bas poids moléculaire, l'acide hyaluronique de haut poids moléculaire a un poids moléculaire supérieur au poids moléculaire de l'acide hyaluronique de bas poids moléculaire.

Lorsque la composition comprend à la fois de l'acide hyaluronique de haut et de bas poids moléculaire, le rapport pondéral acide hyaluronique de haut poids moléculaire / acide hyaluronique de bas poids moléculaire peut aller de 1 000 à 1, notamment de 500 à 2, en particulier de 100 à 5, voire de 50 à 10.

Selon un autre mode de «réalisation, lorsque la composition comprend à la fois de l'acide hyaluronique de haut et de bas poids moléculaire, le rapport pondéral acide hyaluronique de haut poids moléculaire / acide hyaluronique de bas poids moléculaire va de 10 à 0,1, notamment de 5 à 0,2, en particulier de 2 à 0,5, voire est d'environ 1.

Le poids moléculaire peut être mesuré par la classique méthode HPLC élution/exclusion.

Selon un mode de réalisation particulier, la composition comprend comme acide hyaluronique uniquement de l'acide hyaluronique de haut poids moléculaire. Ceci signifie notamment que le rapport pondéral en acide hyaluronique de bas poids moléculaire par rapport à l'acide hyaluronique de haut poids moléculaire est inférieure ou égale à 0,1 %, en particulier inférieur à 0,01 %, voire est de 0.

En particulier, la composition comprend une teneur en acide hyaluronique de haut poids moléculaire supérieure ou égale à 0,1 % en poids, notamment supérieure ou égale à 0,3 % en poids, en particulier supérieure ou égale à 0,4 % en poids, voire supérieure ou égale à 0,5 % en poids par rapport au poids total de la composition.

Dans ce cas, la teneur en acide hyaluronique de haut poids moléculaire peut aller de 0,1 à 2 % en poids, en particulier de 0,2 à 2 % en poids, notamment de 0,2 à 1 % en poids, tout particulièrement de 0,2 à 0,5 % en poids, par rapport au poids total de la composition.

Selon une autre variante, la teneur en acide hyaluronique de haut poids moléculaire peut aller de 0,1 à 1 % en poids, en particulier de 0,1 à 0,5 % en poids par rapport au poids total de la composition. La présente composition à usage topique est dépourvue de conservateurs, notamment chimiques, utilisés seuls ou en association. En particulier, ladite composition est dépourvue de conservateurs choisis parmi la liste des produits autorisés par la réglementation, notamment choisis parmi :
- les ammoniums quaternaires, notamment chlorure de benzalkonium, alkyldiméthylbenzylammonium, cétrimide, chlorure de cétylpyridinium, bromure de benzododécinium, chlorure de benzothonium, chlorure de cetalkonium
- les conservateurs mercuriels, comme nitrate/acétate/borate phénylmercurique, thiomersal,
- les conservateurs alcooliques, comme chlorobutanol, alcool benzylique, phényléthanol, alcool phénylethylique,
- les acides carboxyliques, tels qu'acide sorbique,
- les phénols, en particulier méthyl/propyl parabène, et/ou
- les amidines, par exemple chlorhexidine digluconate.

La composition peut encore être dépourvue d'EDTA en tant que tel ou encore en association avec au moins un conservateur. L'EDTA, étant un agent chélateur potentialisant l'efficacité des conservateurs, en association avec au moins un conservateur.

Par « dépourvue », on entend au sens de la présente invention une teneur inférieure ou égale à 10 ppm, notamment inférieure ou égale à 1 ppm, voire égale à 0 ppm. La composition comprend au moins un agent de cicatrisation qui est un composé chimique accélérant la cicatrisation de la peau et la régénération des cellules.

L'agent de cicatrisation est choisi parmi l'allantoïne, la vitamine A, le sucralfate, qui est un disaccharide polysulfaté, et/ou le saccharose sulfate basique d'aluminium.

L'agent de cicatrisation peut être présent en une teneur supérieure ou égale à 0,01 % en poids, notamment à une teneur supérieure ou égale à 0,1% % en poids, en particulier supérieure ou égale à 0,2 % en poids par rapport au poids total de la composition.

L'allantoïne est un composé chimique azoté d'origine organique ou végétale, produit de l'oxydation de l'acide urique présente des propriétés cicatrisantes et régénérantes. La composition peut comprendre une teneur en allantoïne supérieure ou égale à 0,01 % en poids, notamment à une teneur supérieure ou égale à 0,1 % en poids, en particulier supérieure ou égale à 0,2 % en poids par rapport au poids total de la composition.

La vitamine A, peut être présente sous forme de rétinal, d'acide rétinoïque ou de rétinyl phosphate ou palmitate. La composition peut comprendre une teneur en vitamine A supérieure ou égale à 0,01 % en poids, notamment à une teneur supérieure ou égale à 0,1 % en poids, en particulier supérieure ou égale à 0,2 % en poids par rapport au poids total de la composition.

Selon un mode de réalisation particulier la composition comprend de l'allantoine et de la vitamine A. Dans ce cas, le rapport pondéral allantoine / vitamine A peut aller de 10 à 1, notamment de 2 à 1, en particulier être d'environ 1.

Selon un autre mode de réalisation particulier, la composition comprend un sucralfate, dissacharide polysulfaté, et/ou du saccharose sulfate basique d'aluminium.

La composition peut comprendre au moins un extrait de plante. Celui-ci peut en particulier être choisi parmi les extraits d'alchemille, de lierre, de prêle, de réglisse, de concombre, d'arnica, de Ginkgo biloba, d'échinacée, de pépins de raisin, d'avocat, de camomille, de centella asiatica, de calendula officinalis, d'arnica Montana , de houblon, de romarin, de mauve, de pervenche, de fleur d'oranger, de thé vert, d'aloès vera et leurs mélanges, et en particulier parmi les extraits de centella asiatica, de calendula officinalis, d'arnica Montana et leurs mélanges.

Les extraits de plante peuvent être choisis pour leurs propriétés anti-inflammatoires, cicatrisantes, sédatives, astringentes, apaisantes, adoucissante, anti-oedémateux, anti-oxydantes, régénérantes, hydratantes, antalgiques, antiseptiques, émollientes, protectrice, hémostatiques, anesthésiques, bactéricides et/ou immunomodulatrices.

Les extraits de plantes peuvent être utilisés seuls ou en association.

La composition peut comprendre une teneur totale en extrait végétal supérieure ou égale à 1 % en poids, notamment une teneur supérieure ou égale à 2 % en poids, en particulier supérieure ou égale à 3 % en poids par rapport au poids total de la composition.

La composition comprend une teneur totale en extrait végétal inférieure à 10 % en poids par rapport au poids total de la composition.

La composition comprend un solvant, celui-ci comprend, voire est constitué, d'eau.

L'eau utilisée est de l'eau purifiée ou stérilisée, notamment par filtration stérilisante, en particulier via une filtration à 0,22 µm.

Selon un mode de réalisation particulier, la composition, stérile et/ou décontaminée, avec un dénombrement de la flore aérobie inférieur à 1 CFU/ml, à usage topique comprend, voire consiste en :
- de l'acide hyaluronique à une teneur allant de 0,1 à 2 % en poids, notamment de 0,2 à 2 % en poids ou de 0,1 à 0,5 % en poids, par rapport au poids total de la composition,
- au moins un agent de cicatrisation de la peau choisi parmi :
   ∘ l'allantoïne, en une teneur supérieure ou égale à 0,1 % en poids par rapport au poids total de la composition,
   ∘ la Vitamine A, en une teneur supérieure ou égale à 0,01 % en poids par rapport au poids total de la composition,
   ∘ le sucralfate, et/ou
   ∘ le saccharose sulfate basique d'aluminium,
- éventuellement au moins un extrait de plante, en quantité inférieure à 10% en poids et
- au moins un solvant; ladite composition étant dépourvue de conservateurs.

Outre le solvant, l'acide hyaluronique, l'agent de cicatrisation et les extraits de plante, la composition peut comprendre un nombre limité de constituants supplémentaires. En particulier elle ne comprend que 10, notamment que 8, en particulier que 6, voire que 4, encore plus particulièrement que 2 constituants supplémentaires.

Selon une variante particulière elle ne comprend aucun constituant supplémentaire.

Les constituants supplémentaires sont choisis pour ne pas présenter un terrain favorable à la croissance de micro-organismes.

La solution peut se présenter sous la forme d'une solution, d'une émulsion, d'un gel, ou d'une solution micellaire.

Par « émulsion » on entend un mélange stable et homogène de deux substances liquides non miscibles, en particulier se présentant sous la forme d'une émulsion d'huile dans l'eau (émulsion H/E) ou d'une émulsion d'eau dans l'huile (émulsion E/H)

Par « gel » on entend une solution contenant en suspension des particules suffisamment petites pour que le mélange soit homogène, appelé aussi suspension colloïdale.

L'évaluation de la sensibilité de la formule, ou de la composition, aux germes est effectuée suivant la méthode de Pharmacopée Européenne 6^{ème} édition - 2008 (addendum 6.3) et la réalisation d'un test d'efficacité afin de vérifier la croissance éventuelle d'Aspergillus Niger, des bactéries gram + Staphylococcus Aureus, des levures Candida Albicans et des bactéries gram - Pseudomonas Aeruginosa.

Selon un autre de ses aspects, l'invention a pour objet un ensemble comprenant une composition selon l'invention qui est conditionnée dans un conditionnement étanche.

Ceci peut notamment permettre une conservation satisfaisante de la composition, en particulier une conservation de longue durée et/ou une conservation à température ambiante.

Ledit conditionnement peut encore isoler la composition du milieu extérieur, et en particulier isoler la composition de la lumière.

La solution peut être conditionnée par remplissage dans une zone à atmosphère contrôlée ou contamination réduite, ou par remplissage aseptique en milieu stérile, dans des contenants, notamment des flacons, de type unidose ou monodose, dans des contenants de type multidose permettant de protéger la solution de toute contamination pendant l'utilisation, notamment tels que décrits dans les documents suivants WO 2008/015505, US 2009/0294347, US 2007/0210121, EP 2 085 068, FR 2 816 600, US 5,232,687 ou FR 2 873 358, ou dans tout autre contenant, comme des flacons, destinés à l'administration topique de médicaments, en particulier permettant d'éviter l'usage de conservateurs.

En particulier, ces conditionnements peuvent être équipés de systèmes de délivrance permettant d'éviter une contamination de la composition, notamment en filtrant l'air ambiant susceptible d'entrer dans le conditionnement pendant l'utilisation, ou en fonctionnant sans laisser l'air ambiant entrer.

Tout particulièrement, la composition peut se situer dans une poche souple sur laquelle une pression est effectuée, par exemple via un gaz sous pression, afin de pouvoir délivrer la composition. Ce type de système peut présenter l'avantage d'éviter toute entrée dans le contenant comprenant la composition et ainsi de limiter, voire d'éliminer, les risques de contamination.

Le choix du conditionnement peut être effectué en fonction de sa faible perméabilité à l'oxygène, à l'azote et composés à base d'azote tels que nitrates, nitrites, ammoniaque ainsi que les phosphates, c'est à dire des produits susceptible soit d'oxyder la composition, soit d'être un nutriment des bactéries résiduelles de la composition après traitement.

En particulier, les parois constituant le conditionnement peuvent comprendre des matériaux connus pour leur très faible perméabilité aux gaz, en particulier à l'oxygène et à la vapeur d'eau, comme l'aluminium ou les polymères d'éthylène-alcool vinylique (EVOH), le polychlorure de vinylidène (PVDC), le polyacrylonitrile (PAN) ou le polyamide (PA).

Ces matériaux peuvent être utilisés seuls, ou en combinaison avec d'autres matériaux comme proposé par exemple dans le brevet DE 34 19 255.

Le choix du conditionnement peut également être déterminé par son aptitude à maintenir une étanchéité pendant le traitement stérilisant en température.

Les compositions selon l'invention sont destinées à une utilisation topique, d'une façon générale pour toute utilisation corporelle, et en particulier sur les paupières et les annexes de l'oeil, les mains, les lèvres et le visage.

Elles peuvent être destinées à :
- nettoyer le bord palpébral, ou bord libre de la paupière, ou le visage,
- protéger la peau,
- maintenir l'hydratation de la peau,
- favoriser la cicatrisation, en particulier la cicatrisation dans les suites post-opératoires, et/ou
- participer à la diminution de l'inflammation.

D'une façon générale, les compositions selon l'invention peuvent être utilisées sur les peaux lésées, en particulier du visage, des mains, des pieds, des lèvres ou du corps ayant subi une destruction d'origine diverse, comme une destruction traumatique, infectieuse, tumorale, vasculaire ou iatrogène, et demandant une réparation correspondant à la cicatrisation. Tout particulièrement les compositions selon l'invention permettent d'accélérer celle-ci.

Selon un mode de réalisation particulier, les compositions selon l'invention peuvent être utilisées pour la protection et/ou le nettoyage des paupières et du visage, notamment :
1) afin d'éliminer les squames et les croûtes des paupières pathologiques chez les patients atteints de blépharites,
2) comme cicatrisant dans les suites post-chirurgicales, comme les blépharoplasties, l'ectropion ou les lésions, notamment cancéreuses, et/ou
3) pour la prévention et/ou le traitement de la sécheresse oculaire, en particulier consécutifs du mauvais état général des paupières suite à l'utilisation de traitements médicamenteux, chimiques ou suite à une chirurgie.

Ainsi, l'invention a pour objet l'utilisation topique d'une composition selon l'invention, ou une composition selon l'invention destinée à être utilisée, en tant qu'agent nettoyant du bord palpébral ou du visage, agent protecteur de la peau, agent cicatrisant, en particulier agent cicatrisant dans les suites post-opératoires, et/ou agent anti-inflammatoire.

L'invention a encore pour objet l'utilisation topique d'une composition selon l'invention, ou une composition, notamment pharmaceutique, selon l'invention destinée à être utilisée, en tant que :
- agent permettant l'élimination des squames et des croûtes des paupières pathologiques chez les patients atteints de blépharites,
- agent cicatrisant dans les suites post-chirurgicales, comme les blépharoplasties, et l'ectropion, ou les lésions, notamment cancéreuses, et/ou
- agent de prévention et/ou de traitement de la sécheresse oculaire, en particulier consécutifs du mauvais état général des paupières suite à l'utilisation de traitements médicamenteux, chimiques ou suite à une chirurgie.

L'invention a également pour objet l'utilisation topique d'une composition selon l'invention, ou une composition, notamment pharmaceutique, selon l'invention destinée à être utilisée, en tant qu'agent
- destiné à la protection des mains présentant des gerçures, en particulier en favorisant la cicatrisation,
- destiné à la protection des lèvres très sèches et desquamées présentant des gerçures, en particulier en favorisant l'hydratation et/ou la cicatrisation,
- destiné à l'hydratation et à la protection des peaux lésées ou irrités, notamment par une pathologie, un traitement médicamenteux, par exemple par l'isotrétinoïne, physique, par exemple la dermabrasion, et/ou chimique, comme le peeling, et/ou
- destiné à protéger, hydrater et/ou apaiser les paupières à la suite d'épisodes inflammatoires, comme l'eczéma.

L'invention a encore pour objet une composition pharmaceutique ou un médicament comprenant, ou consistant en, une composition selon l'invention, en particulier telle que définie ci-dessus, et tout particulièrement destinée à prévenir et/ou à traiter une ou plusieurs des indications définies ci-dessus. La composition selon l'invention, ledit médicament ou ladite composition pharmaceutique peuvent être destinés à prévenir et/ou à traiter les indications évoquées ci-dessus, en particulier la sécheresse oculaire et les paupières pathologiques, ou encore être destinés à permettre et/ou à accélérer la cicatrisation, notamment dans les suites post-chirurgicales, comme les blépharoplasties, l'ectropion ou les lésions, notamment cancéreuses.

Selon un autre de ses aspects, l'invention a pour objet un procédé de préparation d'une composition dépourvue de conservateurs comprenant de l'acide hyaluronique, en particulier à une teneur supérieure ou égale à 0,1 % en poids par rapport au poids total de la composition, décontaminée et/ou stérile, dans un conditionnement étanche, comprenant, voire consistant en, les étapes suivantes consistant à :
a) stériliser les articles de conditionnement, en particulier par traitement ionisant ou oxyde d'éthylène,
b) purifier le(s) solvant(s), en particulier lorsqu'il comprend, ou consiste en, de l'eau, par exemple par filtration, en particulier par filtration sur filtre 0,22 µm,
c) lorsqu'au moins un extrait de plante est présent, décontaminer celui-ci, notamment par un traitement ionisant, en particulier par un traitement ionisant à une valeur d'au moins 5 kGy,
d) mélanger les composants de la composition, notamment l'acide hyaluronique, éventuellement au moins un extrait de plante et/ou un composé ayant une action cicatrisante, dans au moins un solvant,
e) conditionner dans un environnement à contamination contrôlée ou stérile la composition dans un conditionnement étanche,
f) chauffer la composition dans son conditionnement étanche, notamment à l'aide de chaleur sèche, dans une étuve à une température allant de 65 à 85°C, pendant au moins une période d'au moins 1h, suivi d'un refroidissement au moins à température ambiante,
g) répéter l'étape f) au moins une fois, notamment au moins le jour suivant, voire les 2 jours suivants, voire les 3 jours suivants, et
h) récupérer le conditionnement étanche comprenant la composition décontaminée et/ou stérile.

Ledit procédé peut permettre d'obtenir une composition dans un contenant étanche comprenant de l'acide hyaluronique, notamment tel que décrit ci-dessus, en particulier dans les quantités et rapport décrits ci-dessus.

Selon un mode de réalisation particulier, le procédé permet d'obtenir une composition selon l'invention.

Le procédé permet notamment l'obtention d'une composition présentant une longue durée de conservation sans qu'il soit requis d'utiliser des conservateurs et/ou de l'EDTA, notamment tels que décrits ci-dessus, tout en étant non destructive pour l'acide hyaluronique.

En particulier ce procédé n'affecte pas ou peu les propriétés dudit acide hyaluronique, en particulier par rapport à son poids moléculaire.

Les composés hydrophiles comme l'acide hyaluronique, l'allantoïne et les extraits de plante peuvent être incorporés dans le solvant jusqu'à totale hydratation.

Les composés hydrophobes peuvent être incorporés dans la solution sous la forme d'émulsions huile dans eau, de liposomes, de micelles, de dendrimères ou autre suspension.

La taille des globules incorporés dans la solution est variable, cependant, dans le cas ou la solution comprenant les émulsions huile dans eau devait être filtrée la taille maximale de globules peut être inférieure ou égale à 220 nm, en particulier inférieure ou égale à 160 nm.

Dans le cas d'une filtration à caractère stérilisant, les globules de l'émulsion doivent pouvoir passer par un filtre de 0,22 µm. Des globules d'une taille supérieure à 220 nm peuvent passer à travers un filtre de 0,22 µm en modifiant temporairement leur forme, cependant ralentissant le procédé de filtration.

La température de chauffage de l'étape f) peut aller de 65 à 85°C, voire aller de 68 à 80°C.

La période de chauffage de l'étape f) peut aller de 1 à 3 h, notamment de 1 à 2 h, en particulier de 1 à 1,5 h, voire être de 1h.

Les périodes de chauffage sont séparées par des périodes dites « TA » où la composition est à température ambiante ou inférieure, par exemple 0°C ou moins. Lesdites périodes TA peuvent aller de 5 à 40 h, notamment de 10 à 30 h, voire de 20 à 25 h.

Tout particulièrement, le chauffage de l'étape f) est effectué sur des périodes séparées d'au moins 5 heures, notamment d'au moins 10h, voire de 24h.

L'étape g) peut comprendre, ou consister en, une à cinq répétitions du chauffage de l'étape f), en particulier 2 à 4 répétitions, voire deux répétitions.

Selon un autre aspect, l'invention a encore pour objet une composition susceptible d'être obtenue par le procédé tel que défini dans la présente description.

En particulier, la décontamination de l'étape c) est effectuée par un traitement par rayons gamma ou beta d'une dose inférieure ou égale à 50 kGy, voire d'une dose inférieure ou égale à 25 kGy, voire d'une dose égale à 5 kGy, de sorte à diminuer la charge microbienne initiale des extraits de plantes, sans altérer leur propriétés.

Avantageusement, le procédé permet d'obtenir une composition stérile tout en affectant au minimum l'acide hyaluronique de départ, en particulier par rapport à son poids moléculaire et/ou son organisation chimique.

En particulier le procédé diminue le poids moléculaire de l'acide hyaluronique de moins de 20 %, notamment de moins de 10 %, en particulier de moins de 5%.

Le choix du poids moléculaire de l'acide hyaluronique de départ, ou de la combinaison d'acides hyaluroniques de différents poids moléculaire sera effectué de sorte à obtenir après traitement par chauffage de l'étape f)-g) le poids moléculaire souhaité, ou la combinaison de poids moléculaires souhaités.

Selon encore un autre de ses aspects, l'invention a pour objet une composition susceptible d'être obtenue, voire directement obtenue, par un procédé tel que défini ci-dessus.

Bien entendu, les différentes caractéristiques exposées dans la présente description peuvent être combinées entre elles.

Les exemples suivants sont donnés à titre illustratif de l'invention.

### EXEMPLES

### Exemple 1

Une composition 1 et une composition 2 comprenant 200 mg d'acide hyaluronique respectivement d'environ 800 kDa et d'environ 500 kDA dans 100 ml d'eau subissent les traitements thermiques décrits dans le tableau 1.

Les acides hyaluroniques utilisés pour ces compositions peuvent être par exemple fourni par FIDIA Farmaceutici S.p.A. sous les références HYALOFTIL, HA 1 000 000 Da ou HA 500 000 Da, ou HTL Biotechnology, fabriqué en fonction du poids moléculaire souhaité.

**Tableau 1**

| Échantillon | Traitement | Poids moléculaire (Da) |
|---|---|---|
| Composition 1 | Sans | 871 400 |
| Composition 1 | 75°C, 15 min | 844 900 |
| Composition 1 | 75°C, 19 min | 856 800 |
| Composition 1 | 80°C, 10 min | 851 200 |
| Composition 2 | Sans | 565 000 |
| Composition 2 | 75°C, 15 min | 553 000 |
| Composition 2 | 80°C, 10 min | 574 000 |

Il ressort de ces essais que le poids moléculaire de l'acide hyaluronique présent dans ces compositions est diminué de moins de 20 %.

### Exemple 2

Trois compositions ont été préparées :
- composition A contenant une concentration de 0,2% d'Acide Hyaluronique de 2 poids moléculaires différents, l'un compris entre 700 et 750 kDa, l'autre compris entre 750 et 800 kDa, dans une proportion de 1:1 dans 100 ml d'eau,
- composition B contenant une concentration de 0,5% d'Acide Hyaluronique de poids moléculaire compris entre 700 et 750 kDa dans 100 ml d'eau, et
- composition C contenant une concentration de 0,5% d'Acide Hyaluronique d'un autre poids moléculaire allant de 750 à 800 kDa dans 100 ml d'eau.

Les 3 compositions ont fait l'objet de 3 différents traitements en température :
- un traitement à 70°C pendant 1 h suivi d'un refroidissement reproduit 1 fois par jour pendant 3 jours,
- un traitement à 100°C pendant 1h suivi d'un refroidissement, et
- un traitement à 121°C pendant 20 min suivi d'un refroidissement.

**Tableau 2**

| Échantillon | Traitement | Poids moléculaire (Da) |
|---|---|---|
| Composition A | Sans | 758 000 |
| Composition A | 70°C, 1h, 3x/24h | 741 000 |
| Composition A | 100°C, 1h, 1x | 615 000 |
| Composition A | 121°C, 20 min, 1x | 204 000 |
| Composition B | Sans | 731 000 |
| Composition B | 70°C, 1h, 3x/24h | 696 000 |
| Composition B | 100°C, 1h, 1x | 398 000 |
| Composition B | 121°C, 20 min, 1x | 291 000 |
| Composition C | Sans | 750 000 |
| Composition C | 70°C, 1h, 3x/24h | 761 000 |
| Composition C | 100°C, 1h, 1x | 692 000 |
| Composition C | 121°C, 20 min, 1x | 400 000 |

Les traitements à 100°C pendant 1h et 121°C pendant 20 min entraînent une dégradation significative du poids moléculaire de l'acide hyaluronique, tandis que le traitement à 70°C pendant 1h suivi d'un refroidissement à température ambiante réalisé 3 fois à 24h d'intervalle n'a pas entraîné de modification significative du poids moléculaire de l'acide hyaluronique. Par ailleurs, dans tous les échantillons ayant subi un traitement thermique, le niveau de propreté microbiologique a été évalué conforme (< 1 cfu/g) et de plus stérile au sens de la Pharmacopée Européenne.

## Revendications

1. Procédé de préparation d'une composition dépourvue de conservateurs comprenant de l'acide hyaluronique décontaminée et/ou stérile dans un conditionnement étanche comprenant les étapes suivantes consistant à :
a) stériliser les articles de conditionnement,
b) purifier le(s) solvant(s),
c) lorsqu'au moins un extrait de plante est présent, décontaminer celui-ci,
d) mélanger les composants de la composition dans au moins un solvant,
e) conditionner dans un environnement à contamination contrôlée et/ou stérile la composition dans un conditionnement étanche,
f) chauffer la composition dans son conditionnement étanche dans une étuve à une température allant de 65 à 85°C, pendant au moins une période d'au moins 1h, suivi d'un refroidissement à température ambiante,
g) répéter l'étape f) au moins une fois, notamment au moins le jour suivant, voire les 2 jours suivants, voire les 3 jours suivants, et
h) récupérer le conditionnement étanche comprenant la composition décontaminée et/ou stérile.

2. Procédé selon la revendication 1 dans lequel l'étape f) est réalisée à une température allant de 68°C à 80°C.

3. Procédé selon l'une des revendications 1 à 2 dans lequel le chauffage de l'étape f) est réalisé pendant une période allant de 1 à 3 heures.

4. Procédé selon l'une des revendications précédentes dans lequel le refroidissement à température ambiante de l'étape f) est réalisé pendant une période allant de 5 à 40 heures.

5. Procédé selon l'une des revendications précédentes dans lequel l'étape g) consiste en une à cinq répétitions de l'étape f).

6. Procédé selon l'une des revendications précédentes dans lequel la composition comprend :
- de l'acide hyaluronique à une teneur supérieure ou égale à 0.1% en poids par rapport au poids total de la composition ;
- au moins un agent de cicatrisation de la peau choisi parmi l'allantoïne, la vitamine A, le sucralfate et le saccharose sulfate basique d'aluminium ;
- éventuellement au moins un extrait de plante, et
- au moins un solvant.

7. Composition à usage topique stérile et/ou décontaminée, avec un dénombrement de la flore aérobie inférieur à 1CFU/ml, absence de pseudomonas aeruginosa, absence de staphylococcus aureus, absence de candida albicans et absence de germe, susceptible d'être obtenue par le procédé selon l'une des revendications 1 à 6 dans laquelle la composition comprend :
- de l'acide hyaluronique à une teneur supérieure ou égale à 0.1% en poids par rapport au poids total de la composition ;
- au moins un agent de cicatrisation de la peau choisi parmi l'allantoïne, la vitamine A, le sucralfate et le saccharose sulfate basique d'aluminium ;
- éventuellement au moins un extrait de plante, en une quantité inférieure à 10% en poids et
- au moins un solvant ;
ladite composition étant dépourvue de conservateurs.

8. Composition à usage topique selon la revendication 7 comprenant de l'acide hyaluronique de haut poids moléculaire et de l'acide hyaluronique de bas poids moléculaire, ledit acide hyaluronique de haut poids moléculaire ayant un poids moléculaire allant de 800 à 1200 kDa et l'acide hyaluronique de bas poids moléculaire ayant un poids moléculaire allant de 10 à 600 kDa, en particulier en un rapport pondéral allant de 1000 à 1.

9. Composition à usage topique selon la revendication 7 comprenant uniquement de l'acide hyaluronique ayant un poids moléculaire allant de 800 à 1200 kDa.

10. Composition à usage topique selon l'une des revendications 7 à 9 comprenant au moins un extrait de plante parmi les extraits d'alchemille, de lierre, de prêle, de réglisse, de concombre, d'arnica, de Ginkgo biloba, d'échinacée, de pépins de raisin, d'avocat, de camomille, de centella asiatica, de calendula officinalis, d'arnica Montana, de houblon, de romarin, de mauve, de pervenche, de fleur d'oranger, de thé vert, d'aloès vera et leurs mélanges, et en particulier parmi les extraits de centella asiatica, de calendula officinalis, d'arnica Montana et leurs mélanges.

11. Composition à usage topique selon l'une des revendications 7 à 10 pour son utilisation en tant qu'agent cicatrisant, et/ou agent anti-inflammatoire.

12. Ensemble comprenant une composition à usage topique selon l'une des revendications 7 à 11 qui est conditionnée dans un conditionnement étanche par le procédé défini à l'une quelconque des revendications 1 à 6.

13. Utilisation topique cosmétique non-thérapeutique d'une composition telle que définie selon l'une quelconque des revendications 7 à 10, en tant qu'agent nettoyant du bord palpébral ou du visage, agent protecteur de la peau, agent hydratant et/ou agent destiné à la protection des mains ou des lèvres très sèches et desquamées, présentant des gerçures.

## Patentansprüche

1. Verfahren zur Herstellung einer konservierungsmittelfreien Zusammensetzung, die dekontaminierte und/oder sterile Hyaluronsäure umfasst, in einer dichten Verpackung, das die folgenden Schritte umfasst:
a) Verpackungsartikel sterilisieren,
b) Lösungsmittel reinigen,
c) wenn mindestens ein Pflanzenextrakt vorhanden ist, diesen dekontaminieren,
d) die Komponenten der Zusammensetzung in zumindest einem Lösungsmittel mischen,
e) die Zusammensetzung in einer Umgebung mit kontrollierter und/oder steriler Kontaminierung in eine dichte Verpackung verpacken,
f) die Zusammensetzung in ihrer dichten Verpackung in einem Ofen bei einer Temperatur von 65 bis 85 °C mindestens 1 Stunde lang erhitzen und anschließend auf Raumtemperatur abkühlen lassen,
g) den Schritt f) mindestens einmal wiederholen, insbesondere mindestens am Folgetag und sogar an den 2 Folgetagen oder sogar 3 Folgetagen, und
h) die dichte Verpackung mit der dekontaminierten und/oder sterilen Zusammensetzung zurückholen.

2. Verfahren nach Anspruch 1, wobei der Schritt f) bei einer Temperatur von 68 °C bis 80 °C durchgeführt wird.

3. Verfahren nach einem der Ansprüche 1 bis 2, wobei die Erhitzung im Schritt f) über einen Zeitraum von 1 bis 3 Stunden durchgeführt wird.

4. Verfahren nach einem der vorstehenden Ansprüche, wobei die Abkühlung des Schritts f) auf Raumtemperatur über einen Zeitraum von 5 bis 40 Stunden durchgeführt wird.

5. Verfahren nach einem der vorstehenden Ansprüche, wobei der Schritt g) aus einer bis fünf Wiederholungen des Schritts f) besteht.

6. Verfahren nach einem der vorstehenden Ansprüche, wobei die Zusammensetzung umfasst:
- Hyaluronsäure mit einem Gehalt höher oder gleich 0,1 Gew.-%, bezogen auf das Gesamtgewicht der Zusammensetzung;
- mindestens ein Vernarbungsmittel für die Haut, das aus Allantoin, Vitamin A, Sucralfat und basischem Saccharose-Aluminiumsulfat ausgewählt wird;
- eventuell mindestens einen Pflanzenextrakt, und
- mindestens ein Lösungsmittel.

7. Zusammensetzung zum topischen Verwendung sterile und/oder dekontaminierte mit einer Bestimmung der aeroben Flora von unter 1 CFU/ml, Fehlen von Pseudomonas aeruginosa, Fehlen von Staphylococcus aureus, Fehlen von Candida albicans und Fehlen von Keimen, die durch das Verfahren nach einem der Ansprüche 1 bis 6 erzielt werden kann, wobei die Zusammensetzung umfasst:
- Hyaluronsäure mit einem Gehalt höher oder gleich 0,1 Gew.-%, bezogen auf das Gesamtgewicht der Zusammensetzung;
- mindestens ein Vernarbungsmittel für die Haut, das aus Allantoin, Vitamin A, Sucralfat und basischem Saccharose-Aluminiumsulfat ausgewählt wird;
- eventuell mindestens einen Pflanzenextrakt in einer Menge unter 10 % Gew.-% und
- mindestens ein Lösungsmittel;
wobei die Zusammensetzung frei von Konservierungsmitteln ist.

8. Zusammensetzung zum topischen Verwendung nach Anspruch 7, die Hyaluronsäure mit hohem Molekulargewicht und Hyaluronsäure mit niedrigem Molekulargewicht umfasst, wobei die Hyaluronsäure mit hohem Molekulargewicht ein Molekulargewicht im Bereich von 800 bis 1200 kDa aufweist und wobei die Hyaluronsäure mit niedrigem Molekulargewicht im Bereich von 10 bis 600 kDa bewegt, und insbesondere in einem Gewichtsverhältnis im Bereich von 1000 bis 1.

9. Zusammensetzung zum topischen Verwendung nach Anspruch 7, die nur Hyaluronsäure mit einem Molekulargewicht im Bereich von 800 bis 1200 kDa aufweist.

10. Zusammensetzung zum topischen Verwendung nach einem der Ansprüche 7 bis 9, die mindestens einen Pflanzenextrakt aus Alchemilla, Efeu, Schachtelhalm, Süßholz, Gurke, Arnika, Ginkgo biloba, Echinacea, Traubenkernen, Avocadokernen, Kamille, Centella asiatica, Calendula officinalis, Arnika Montana, Hopfen, Rosmarin, Malve, Immergrün, Orangenblüten, grüner Tee, Aloe Vera und Mischungen daraus, und insbesondere aus Centella asiatica, Calendula officinalis, Arnica Montana und Mischungen daraus umfasst.

11. Zusammensetzung zum topischen Verwendung nach einem der vorstehenden Ansprüche 7 bis 10 zur Verwendung als Vernarbungsmittel und/oder entzündungshemmendes Mittel.

12. Packung, umfassend eine Zusammensetzung zum topischen Verwendung nach einem der vorstehenden Ansprüche 7 bis 11, die in einer dichten Verpackung nach dem in einem der Ansprüche 1 bis 6 definierten Verfahren verpackt ist.

13. Topische kosmetische und nicht therapeutische Verwendung einer Zusammensetzung, wie sie nach einem der Ansprüche 7 bis 10 definiert ist, als Reinigungsmittel für den Lidrand oder des Gesichts, als Hautschutzmittel, feuchtigkeitsspendendes Mittel und/oder Mittel für den Schutz von sehr trockenen und schuppigen Händen oder Lippen, die Risse aufweisen.

## Claims

1. A method for preparing a preservatives free composition comprising decontaminated and/or sterile hyaluronic acid in a hermetic packaging comprising the following steps of:
a) sterilizing the packaging items,
b) purifying the solvent(s),
c) when at least one plant extract is present, decontaminating said plant extract,
d) mixing the components of the composition in at least one solvent,
e) packaging in a sterile and/or contamination-controlled environment the composition in a hermetic packaging,
f) heating the composition in its hermetic packaging in an oven at a temperature ranging from 65 to 85 °C, for at least a period of at least 1 hour, followed by cooling at room temperature,
g) repeating step f) at least once, notably at least the following day, or even the following 2 days, or even the following 3 days, and
h) recovering the hermetic packaging comprising the decontaminated and/or sterile composition.

2. The method according to claim 1 wherein the heating temperature of step f) ranges from 68 °C to 80 °C.

3. The method according to any one of claims 1 to 2 wherein the heating period of step f) ranges from 1 to 3 hours.

4. The method according to any one of the preceding claims wherein the cooling at room temperature of step f) lasts for a period ranging from 5 to 40 hours.

5. The method according to any one of the preceding claims wherein step g) consists of 1 to 5 repetitions of step f).

6. The method according to any one of the preceding claims wherein the composition comprises:
- hyaluronic acid at a concentration greater than or equal to 0.1 % by weight in relation to the total weight of the composition;
- at least one skin cicatrization agent selected from the group consisting of allantoin, vitamin A, sucralfate and basic aluminum saccharose sulfate;
- optionally at least one plant extract, and
- at least one solvent.

7. A decontaminated and/or sterile composition for topical use, with an enumeration of the aerobic flora less than 1 CFU/ml, absence of pseudomonas aeruginosa, absence of staphylococcus aureus, absence of candida albicans and absence of bacteria, obtainable by the method according to any one of claims 1 to 6 wherein the composition comprises:
- hyaluronic acid at a concentration greater than or equal to 0.1 % by weight in relation to the total weight of the composition;
- at least one skin cicatrization agent selected from the group consisting of allantoin, vitamin A, sucralfate and basic aluminum saccharose sulfate;
- optionally at least one plant extract, in an amount less than 10 % by weight and
- at least one solvent;
said composition being preservatives free.

8. The composition for topical use according to claim 7 comprising high molecular weight hyaluronic acid and low molecular weight hyaluronic acid, said high molecular weight hyaluronic acid having a molecular weight of 800 to 1200 kDa and the low molecular weight hyaluronic acid having a molecular weight of 10 to 600 kDa, in particular in a weight ratio from 1000 to 1.

9. The composition for topical use according to claim 7 comprising only hyaluronic acid having a molecular weight of 800 to 1200 kDa.

10. The composition for topical use according to any one of claims 7 to 9 comprising at least one plant extract selected from the group consisting of extracts of alchemilla, ivy, horsetail, liquorice, cucumber, arnica, Ginkgo biloba, echinacea, grape seed, avocado, chamomile, centella asiatica, calendula officinalis, arnica Montana, hop, rosemary, mallow, periwinkle, orange blossom, green tea, aloe vera and mixtures thereof, and in particular from extracts of centella asiatica, calendula officinalis, arnica Montana and mixtures thereof.

11. The composition for topical use according to any one of claims 7 to 10 for use as a cicatrization agent and/or an anti-inflammatory agent.

12. A unit comprising a composition for topical use according to any one of claims 7 to 11 which is packaged in a hermetic packaging by the method defined in any one of claims 1 to 6.

13. Non-therapeutic cosmetic topical use of a composition as defined in any one of claims 7 to 10, as a cleaning agent for the palpebral edge or the face, a skin protectant, a hydrating agent and/or an agent for protecting very dry and chapped hands or lips.
